# EUROPEAN PATENT SPECIFICATION

(11) **EP 2 976 107 B1**
(45) Date of publication and mention of the grant of the patent: **20.02.2019**
(21) Application number: 14710921.9
(22) Date of filing: 19.03.2014
(51) Int. Cl.: A61K 47/06, A61K 39/36, A61K 39/39, A61K 9/107, A61K 39/35

(54) **ALLERGEN PREPARATION**
ALLERGENPRÄPARAT
PRÉPARATION ALLERGÉNIQUE

(30) Priority: 19.03.2013 EP 13160006
(43) Date of publication of application: 27.01.2016
(73) Proprietor: ASIT BioTech S.A., 1200 Bruxelles (BE)
(72) Inventor: LEGON, Thierry, B-3360 Bierbeek (BE)
(74) Representative: dompatent von Kreisler Selting Werner - Partnerschaft von Patent- und Rechtsanwälten mbB
(86) International application number: PCT/EP2014/055516
(87) International publication number: WO 2014/147131

(56) References cited:
- WO-A1-2008/000783
- WO-A1-2009/083589
- WO-A1-2012/172037
- WO-A2-99/25823
- US-A1- 2003 129 251
- US-A1- 2004 136 948
- US-A1- 2004 235 774
- FOX CHRISTOPHER B: "Squalene emulsions for parenteral vaccine and drug delivery.", MOLECULES (BASEL, SWITZERLAND) 2009, vol. 14, no. 9, 2009, pages 3286-3312, XP002725808, ISSN: 1420-3049, DOI: 10.3390/molecules14093286

## Description

The present invention is related to an allergen preparation.

Common allergens are pollens, house dust mites, moulds, drugs, foods and animal hair and dander.

The most common allergy diseases are rhinitis, asthma and atopic dermatitis. Allergic asthma is a chronic inflammatory disorder. Symptomatic treatment of allergic disorders is effected by use of antihistaminis, β-antagonists and corticosteroids.

Furthermore, the so called "specific" immunotherapy is based on a hyposensitization. Typically, patients are administered with subcutaneous injection of the specific offending allergens. Treatment is started with small allergen doses and the doses are increased. Treatment is typically maintained for several years. This type of treatment suffers from poor patient compliance and has been questioned due to safety reasons because a patient can suffer from severe anaphylactic reactions.

In addition to methods comprising repeated subcutaneous injections there are also oral hyposensitization methods.

US 4,822,611 discloses a method for treating allergies comprising oral treatment with allergens. It describes the use of commercially available "bulk" allergenic extracts showing batch-to-batch variation and differences in extracts from different manufactures. The preparation of these extracts is not described.

GB 1 247 614 discloses a method of extracting an allergen. The aim of this method is to have a more complete and effective allergenic extract by including all extractable components of the allergen.

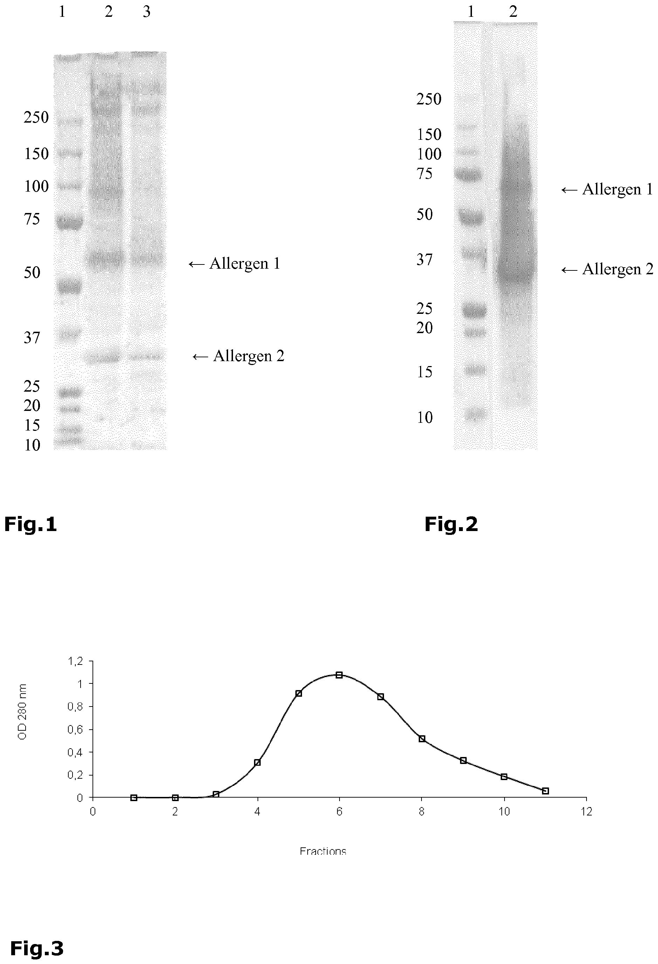

US 5,770,698 discloses a process for purifying extracts of allergenically active proteins. The spectrum of figure 2 of US '698 does not present a peak at 280nm. This implies that the extract contains significant amount on non-protein impurities.

WO 99/22762 discloses a similar method; therefore, the product comprises large amounts of non-protein impurities, too.

WO 99/25823 discloses a preparation comprising a recombinant allergen.

US 2003/0129251 discloses a composition comprising IMP/MC complexes as adjuvant.

US 2004/0235774 discloses the administration of an immune stimulatory nucleic acid, optionally in combination with an asthma/allergy medicament.

Christopher B. Fox in Molecules, 14 (2009) 3286-3312 discloses squalene emulsions for parental vaccine and drug delivery.

US 2004/136948 discloses preparations comprising plant recombinant allergens.

On the other hand, there has been a tendency to develop highly specific preparations based on single epitopes. For example, WO 00/58349 discloses an isolated and purified peptide comprising a leucin positioned two peptide bonds away from a tyrosine/arginine pair. These peptides can be used to prepare a pharmaceutical composition to accomplish treatment or prophylaxis, in this case especially directed to allergy in dogs.

On the one hand, methods are used to purify a specifically identified single allergenic molecule. On the other hand, people are trying to produce allergenic extracts as complete as possible.

According to the first alternative, it is always possible that the allergen preparation lacks the relevant epitopes to induce tolerance in a determined patient. The second alternative has a drawback of batch-to-batch variability and of the presence of compounds able to trigger immune response like DNA molecules, carbohydrates, lipids of complexes thereof.

WO 2008/000793 describes a method of purifying allergens overcoming at least some of the drawbacks of prior art, especially to provide antigens from natural allergens with a significant reduced capability to trigger allergenicity reaction compared to the crude allergen extract but able to stimulate T-cells as well.

WO 2012/172037 discloses a method for the production of hydrolyzed allergens.

Despite significant progress, there is still a need to improve immunogenicity of allergen preparations, especially in the treatment of allergies.

It is an object of the present invention to provide allergen preparations having an improved immunogenicity and their use for the therapeutical applications for treating allergy.

The problem is solved by the provision of an allergen preparation comprising at least one allergen in an oil-in-water emulsion as defined in claim 1.

Oil-in-water emulsions are known in the art in the context of vaccine preparations; see WO 95/17210 and WO 2008/043774 and references cited therein.

It has been found, that adjuvants may improve the efficiency of an allergen preparation, thereby reducing the necessary amount of the allergen in the preparation. This improves safety. It has further been discovered that adjuvants like Al(OH)₃ are not able to bind all allergen proteins/peptides in a similar way. That may negatively influence the efficiency in that some allergens show an improved stimulation of T-cells and B-cells, whereas others show a reduced stimulation. This problem is especially important, if not only a single purified allergen, but a mixture a allergenic proteins or hydrolysates of allergenic proteins is used.

An embodiment of the invention is an allergen preparation comprising an allergen in an oil-in-water emulsion as defined in claim 1..

The allergen preparation comprises squalene, water and one or more surfactants.

Preferably, the one or more surfactants surfactants is selected from the group consisting of Tween 80, CAMPUL POE-O low PV surfactant, SOLITOL HS15 surfactant, PLURONIC F68 block co-polymer, sodium cholate, glycerodeoxy cholate, sphingomyelin, sphingosine, 1,2-dimyristoyl-sn-glycero-3-phosphoethanolamine, L-a-phosphatidylethanolamine, 1,2-dipalmitoyl-sn-glycero-3phosphocholine and egg phosphatidyl choline, sorbitan trioleate or a mixture thereof.

Preferably, the concentration of the allergen is 0.1 to 200 µg/ml in the preparation (at 25°C).

Preferably, the oil content of the preparation is 1 to 10% (w/w) of the preparation.

The amount of the at least one surfactant is preferably 1 to 30 (w/w) of the oil.

Preferred examples of o/w emulsions are e.g.
5 wt-% squalene
0.5 wt-% polysorbate
0.5 wt-% sorbitan trioleate
in aqueous citrate puffer at pH 6.5.

In a further embodiment, the o/w emulsion comprises at least squalene, an aqueous solvent and a polyoxyethylene alkyl ether.

In one embodiment, the o/w emulsions are prepared free of allergens. A solution comprising the allergen is then combined with the o/w emulsion at a ratio of 1:9 to 9:1 (w/w).

The allergen is a hydrolyzed allergen extract.

The method for the production of the allergen extract of the present invention comprises the steps of
a) extracting a source of allergens comprising allergenic proteins to form an extract,
b) purifying the extract to remove non-protein components to form a purified extract,
c) denaturing the purified extract with a first denaturing agent to form a purified denatured extract,
d) refining the purified denatured extract to remove impurities to form a refined denatured extract.

In some embodiments, the method is followed by
- denaturing the refined denatured extract with a second denaturing agent to form denatured allergen mixture.

After the step d), a second denaturing step may be performed. For this denaturing step a second denaturing agent is used which can be the same or have different composition from step c). In a preferred embodiment the reducing agent used for the second denaturation step is TCEP.

It is preferred that the pH for the second denaturing step is set between 1.5 and 9.0. In a preferred embodiment the pH is lower than 7.0 or lower than 5.0 or lower than 3.0 but preferably higher than 1.0. Denaturing is preferably performed for at least 15 minutes, preferably at least 30 minutes and more preferably at least 60 minutes at a temperature between 15 and 40°C, preferably between 20 and 37°C.

In contrast to the methods of prior art, the methods produce allergen extracts which comprise predominantly proteins without purifying the extract to a single peptide or protein.

In contrast to the products of prior art the products of the invention have following advantages:
- Immunogenic substances other than proteins are substantially removed
- The natural allergen extract is able to stimulate T-cells and/or B-cells with the reduced ability to trigger immediate allergic reaction (basophile activation, mast cell degranulation)

As starting materials, different natural occurring allergens can be used. Typical natural starting materials are milk, venom, egg, weed, grass, tree, shrub, flower, vegetable, grain, fungi, fruit, berry, nut, seed, bean, fish, shellfish, seafood, meat, spices, insect, mite including house dust mite, mould, animal, pigeon tick, worm, soft coral, animal dander, nematode, Hevea brasiliensis, and mixtures thereof.

Preferred allergens used in this invention are especially grass pollen, house dust mite, ragweed pollen, cow milk, egg white and peanuts. Preferably, the peanuts are selected among the *Arachis* genus, preferably from *hypogaea* species, more preferably from *hypogaea* and *fastigiata.* Sub-species comprise *Virginia, Spanish, Valencia* varieties and/or hydrids such as *Runner* or even transgenic peanuts obtained by genetic engineering. Preferably, a mixture of at least 2, preferably 3 species/sub-species/varieties/hybrids and/or transgenic peanuts is used. In a preferred embodiment the red seed coat (tegument) of the peanuts has been removed.

Alternatively, synthetic sources of allergens as starting materials can be used. Synthetic sources of allergens means biotechnological produced proteins like recombinant proteins and/or genetically modified organisms.

Preferably, the source comprises a mixture of allergens.

The allergen preparation of the present invention can be used for the preparation of a pharmaceutical composition and/or food composition for inducing tolerance and desensitization. Induction of tolerance can be used to cure or prevent allergic reactions.

The allergic reaction to be treated or prevented depends on the source of allergens, i.e. allergy to peanuts are prevented or treated by using allergens from peanuts, whereas allergy to grass pollen are treated with allergens from grass pollen.

After extraction of the material, the extract is purified to remove non-protein components such as sugars, lipids, nucleic acids and the like. Typical, several different proteins are present in the protein fraction of the purified extract.

According to prior art, one protein is purified and the other remaining proteins are "impurities".

In contrast thereto, it is the aim of the present invention to purify the proteins together. The relative amounts of the proteins in the purified extract can be easily measured using methods like SDS-PAGE followed by densitometry.

For 60% of total weight of the proteins, it is necessary to count the two most dominant proteins at least, i.e. no single protein is 60% (w/w) or more of all proteins. More preferably, 60% of all proteins are formed by the at least 3 dominant proteins, preferably by the at least 4 dominant proteins and more preferably by at least 5, 6, 7, 8, 9 or 10 proteins.

For example, there are the following proteins:

| | |
|---|---|
| Protein 1: | 27% |
| Protein 2: | 13% |
| Protein 3: | 34% |
| Protein 4: | 19% |
| Protein 5: | 17% |

The most dominant proteins forming together at least 60% (≈ 60% or more) are proteins 3+1 (34+27=61%).

Furthermore, the total protein content of the purified extract is at least 60% by weight; preferably the content is at least 70% by weight or 80% by weight, more preferably 90% by weight of the purified extract.

Extraction is preferably performed with aqueous solutions. Suitable salts are salts such as but not restricted to carbonate, bicarbonate, phosphate, acetate, TRIS and HEPES.

Also in contrast to many other extraction methods, it is preferred that the amount of extraction medium is comparatively large, i.e. at least 20 times the weight of the natural source of allergens, preferably 100 time the weight or more.

Purifying of the extract may be performed by one or more of the following:
- ion exchange chromatography steps (including anion exchange chromatography and cation exchange chromatography),
- size exclusion chromatography step (also called gel filtration),
- precipitation steps,
- hydrophobic interaction chromatography steps,
- pseudo-affinity and affinity chromatographies and/or
- diafiltration.

In a preferred embodiment ion exchange chromatography is used wherein in case of a cation exchanger the loading solution has a pH between the pKa of the acidic function of the cation exchanger and the pKa of the protein having the lowest pKa of the proteins in the extract. In case of an anion exchanger the pH is between the pKa of the basic function of the anion exchanger and the pKa of the protein having the highest pKa of the proteins constituting the extract.

Through this method all proteins bind to the ion exchanger while the neutral impurities and the impurities with the same charge as the ion exchange resin will be removed.

In a preferred embodiment, at least one purification step is performed with a solution comprising one or more of a tenside and/or a denaturing agent. The tenside may be non-ionic, anionic, cationic or amphoteric. Suitable denaturing agents are chaotropic agents, reducing agents and mixtures thereof. Suitable denaturing agents are for example urea, guanidinium chloride, ethylene glycol, isopropanol. A suitable concentration of urea is 3 M or more, preferably 4 M or more. A suitable concentration of guanidinium is preferably 2 M, preferably 3 M or more. A suitable concentration of ethylene glycol and/or isopropanol is 5% or more, more preferably 10% or more, up to 20% by weight.

In some cases, the production of the purified extract is sufficient. Extracts of this type may be used to produce *ex vivo* / *in vivo* and *in vitro* diagnostics, prophylactic and therapeutic treatment of allergic diseases.

In some embodiments, the method is further comprises a step of
- hydrolysing the denatured allergen to form an allergen hydrolysate.

This step may follow after denaturation (first or second) or after refining.

It could be shown that some allergens show better hydrolyzation after a two step denaturation.

The advantages of the product obtained thereby are that the peptides are the digestion result of denatured proteins.

In some embodiments, the hydrolysis is followed by
- purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da
   said purified denatured extract comprising proteins, wherein the most abundant (w/w) proteins, forming together at least 60% (w/w) of all proteins, are at least two proteins, and all proteins represent at least 60% (w/w) of the dry weight of the purified denatured extract.

Due to a specified size calibration they have a reduced potency to induce immediate allergic reaction and pro-inflammatory reaction as well.

Denaturing, if necessary is preferably performed in the presence of chaotropic agents, reducing agents or mixtures thereof. Suitable chaotropic agents are for example urea and guanidinium chloride. Typical reducing agents are for example dithiotriethol, β-mercaptoethanol, thio-glycerol and mixtures thereof.

The hydrolysing step is typically performed with an enzyme. Suitable enzymes are for example pepsin, trypsin, chymotrypsin. This hydrolyzing step can be performed in the presence of a chaotropic agent, preferably urea or guanidinium chloride, too. During hydrolysing the concentration of urea and guanidinium chloride should be below 4 M, preferably below 3M.

The hydrolyzing step can also be performed in presence of a reducing agent, preferably TCEP. During hydrolysis, the concentration of TCEP is preferably below 10 mM. Preferably, pepsin is used. More preferably, pepsin at a pH range of 1.0 - 3.0 is used.

In the size calibration step, peptides with a molecular weight larger than 10,000 Da or smaller than 1,000 Da, are removed to some extent.

The peptides of the purified hydrolysate, therefore, comprise peptides with a molecular weights between 1,000 and 10,000 Da. Suitable methods for removing large or small peptides are ultrafiltration and size exclusion chromatography. Again this size exclusion chromatography may be performed in the presence of a chaotropic agent, for example urea, guanidinium chloride, ethylene glycol, isopropanol and mixtures thereof.

Preferably, less than 10% of the peptides have a molecular weight above 10.000 Da and less than 20% of the peptides have a molecular weight below 1.000 Da so that 70%, or more preferably 80% of the peptides are between 10.000 Da and 1.000 Da.

One advantage of the hydrolysate is that the peptides are the digestion result of purified denatured proteins. They have a reduced potency to induce immediate allergic reactions and co-inflammatory reactions as well.

A further disclosure is an allergen extract obtainable by the method of the present invention. Typically also in this extract the most dominant proteins by weight, which form together at least 60% by weight of all the proteins, are at least 2 proteins, preferably at least 3 or 4 proteins or more preferred at least 5, 6, 7, 8, 9 or 10 proteins. The purity is seen by a Optical Density 260 nm:Optical Density 280 nm-ratio of < 1, preferably < 0.9, more preferably between 0.75 and 0.9.

A further disclosure is an allergen hydrolysate obtainable by the method. It can be used for
- *in vivo* diagnosis of allergic diseases: prick tests, intracutaneous injections, conjunctival, sniff and inhalation tests
- *ex vivo* and *in vitro* diagnosis of allergic diseases: ELISA kits or standards to be used in tests
- Prophylatic and therapeutic treatments of allergic diseases: vaccine for desensitization/hyposensitization treatments and modulation of immune response with/without adjuvant combination.

The allergen extract can be used for the preparation of a pharmaceutical composition and/or food composition for inducing tolerance. Induction of tolerance can be used to cure or prevent allergic reactions.

An embodiment of the present invention is a pharmaceutical composition comprising the allergen preparation of the present invention.

Additionally, pharmaceutical composition may comprise one or more of the following substances: nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, dithiothreitol, adrenergic receptor agonists such as salbutanol, adrenergic receptor antagonists such as butoxamine, compounds that regulate the expression of the adhesion molecule ICAM-1, N-acetyl-L-cysteine, y-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, vaccine adjuvant like CpG or MPL or tolerogenic adjuvant like zymosan, beta-1,3-glucan, regulatory T-cell inducer, a muco-adhesive agent for attaching the particle to the intestinal mucosal lining such as a plant lectin, zinc, zinc salts, polysaccharides, vitamins and bacterial lysates.

"Extracting" as used herein is a treatment of an allergen source with an extraction medium including water, buffer or organic solvents to separate soluble ingredients from a non-soluble residue. The use of aqueous systems (comprising at least 50% H₂O) is preferred.

"Denaturing" as used herein is a process in which the proteins lose their quaternary, tertiary and secondary structure, especially this term refers to the treatment with one or several denaturing agents.

A further embodiment of the present invention is a pharmaceutical composition comprising the allergen preparation of the present invention. Additionally, the pharmaceutical composition may comprise one or more of the following substances: nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, reducing agent, dithiothreitol, adrenergic receptor agonists such as salbutanol, adrenergic receptor antagonists such as butoxamine, compounds that regulate the expression of the adhesion molecule ICAM-1, N-acetyl-L-cysteine, y-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, vaccine adjuvant or immunomodulators like CpG, aluminum hydroxide, calcium phosphate, TLR-4 agonists (i.e. MPL) and TLR-9 agonists or tolerogenic adjuvant like zymosan, beta-1,3-glucan, regulatory T-cell inducer, a muco-adhesive agent for attaching the particle to the intestinal mucosal lining such as a plant lectin, zinc, zinc salts, polysaccharides, vitamins and bacterial lysates or particles displaying surface linked antibodies.

In a preferred embodiment, the pharmaceutical composition is prepared for subcutaneous administration, nasal administration, epicutaneous administration, intralymphatic administration, oral administration, for sublingual drug delivery, or for enteric drug delivery.
Figure 1: Immunoreactivity by IgG western-blot. Lane1 : molecular weight markers, lane 2 : crude grass pollen protein extract, lane 3 : purified allergen denatured extract. Membrane blocked by BSA 5 % and milk 3%. Patient serum diluted to 1/250. IgG binding was detected by goat anti-human IgG HRP diluted to 1/2,500 and revealed by TMB substrate. Allergen 1 : ± 61-54 kDa, Allergen 2: ± 36-31 kDa.
Figure 2: Immunoreactivity by IgE western-blot. Lane1 : molecular weight markers, lane 2 purified grass pollen proteins. Membrane blocked by BSA 5 % and milk 3%. Patient serum diluted to 1/5. IgE binding detected by goat anti-human IgE HRP diluted to 1/10,000 and revealed by TMB substrate. Allergen 1: ± 61-54 kDa Allergen 2: ± 36-31 kDa.
Figure 3: Exclusion peak of SEC G25 elution profile. The ratio column volume / sample volume was 12. The resin was equilibrated with Tris.HCl 25 mM, urea 1.5 M, pH 8.0 at a flow rate of 9 ml/min. The elution was followed by the absorbance at 280 nm.

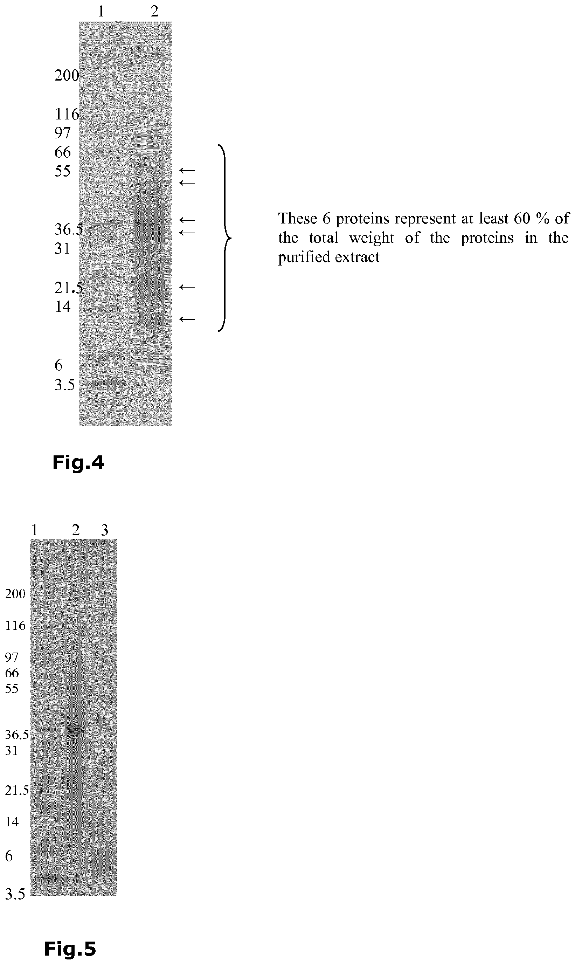

Figure 4: Protein profile by SDS-PAGE. 4 - 12% Bis-Tris gel. Lane 1 : molecular weight markers, lane 2 : purified grass pollen allergen denatured extract. Staining performed with Coomassie brillant blue R-250.
Figure 5: Protein and peptide profiles by SDS-PAGE. 4 - 12% Bis-Tris gel. Lane 1 : molecular weight markers, lane 2 : purified grass pollen allergen denatured extract (13 µg), lane 3 : hydrolysate (13 µg). Staining performed with Coomassie brillant blue R-250.

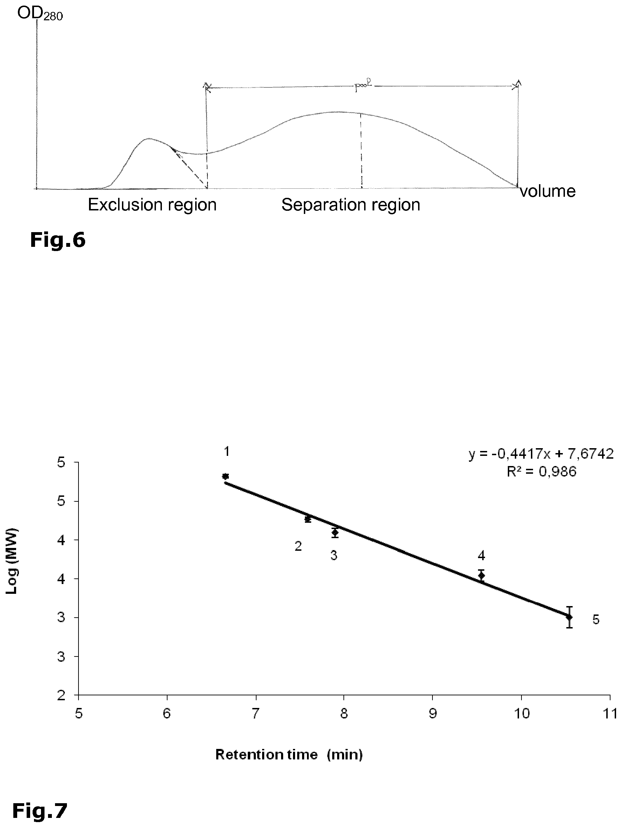

Figure 6: G50 SEC elution profile. The column was equilibrated with urea 2 M, NaCl 100 mM, pH 3.0. Flow rate 15 ml/min. The ratio column volume / sample volume was 10. The elution was followed by the absorbance at 280 nm.
Figure 7: Calibration curve for HPLC analysis. 10 µl of the following standards (1 mg/ml) were injected onto the BioSep-SEC S2000 column: 1. Bovine Serum Albumin (66 kDa), 2. β-Lactoglobulin (18.5 kDa), 3. Cytochrome C (12 kDa), 4. Glucagon (3.5 kDa), 5. 1 kDa synthetic peptide.

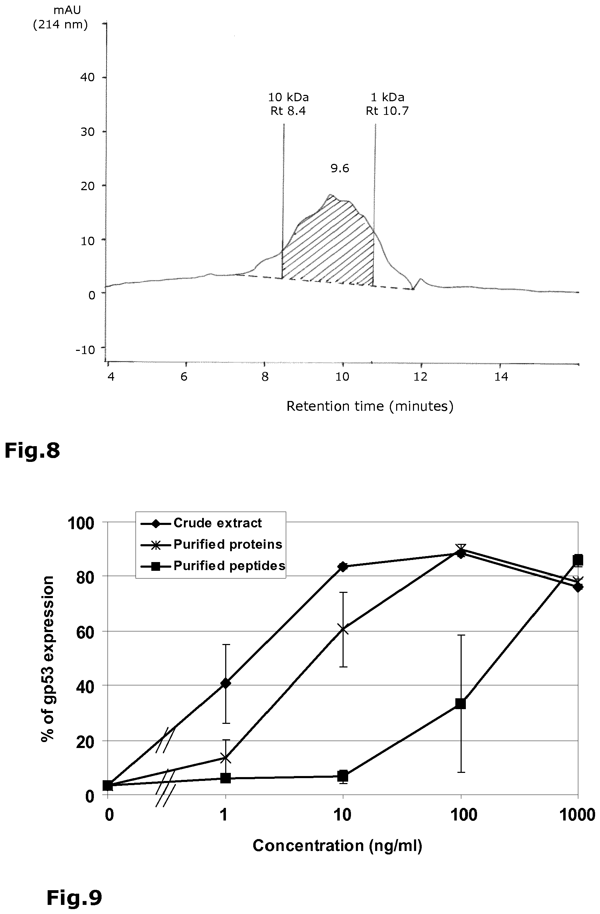

Figure 8: Size exclusion HPLC profile. Column : BioSep-SEC S2000 (PHENOMENEX). Elution buffer : Na₂HPO₄ 50 mM - SDS 0.5% (w/v) pH 6.8. Flow rate 1 ml/min. Detection at 214 nm. 10 µl of the samples were injected. The area under the curve, between 10 kDa and 1 kDa limits was used to calculate the percentage of the peptides of interest.
Figure 9: Allergenicity properties of the pollen-derived products. Blood samples from pollen allergic volunteers were incubated with increasing concentrations (0, 1, 10, 100 and 1000 ng/ml) of either pollen crude extract, pollen purified proteins and pollen purified peptides. gp53 protein expression was measured by flow cytometry with gating on IgE-positive leukocytes. Results are expressed as % of gp53 positive cells in activated cells (mean ± deviation of 2 determinations).
   The method of the present invention is further exemplified by the following, nonlimiting examples.

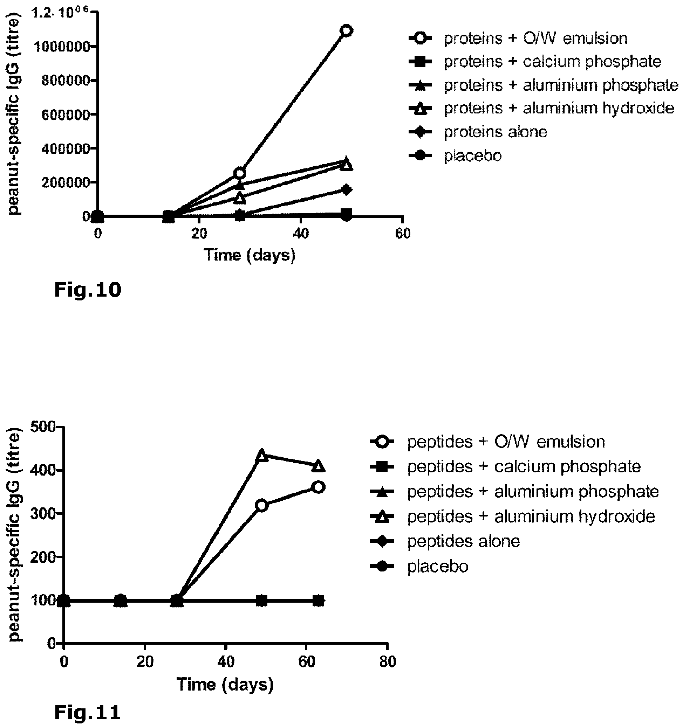

Figure 10: Evolution of peanut-specific IgG titres in serum of mice treated with 25 µg of peanut proteins alone or in combination with oil-in-water emulsion (O/W emulsion), calcium phosphate, aluminium phosphate, aluminium hydroxide. A control group was injected with the placebo. The results represent medians of peanut-specific IgG titres of each group (n=10).
Figure 11: Evolution of peanut-specific IgG titres in serum of mice treated with 100 µg of peanut peptides alone or in combination with oil-in-water emulsion (O/W emulsion), calcium phosphate, aluminium phosphate, aluminium hydroxide. A control group was injected with the placebo. The results represent medians of specific IgG titres of each group (n=10).

### Examples

### Example 1: Extraction

1% (w/v) pollen (*Lolium perenne* from ALLERGON) was added to sodium bicarbonate (12.5 mM) and incubated 2 h under stirring. The solution was then clarified and filtrated by adding celite (ACROS) at 2% (w/v) and passing through a 0.2 µm filter. This sample constitutes the crude extract.

The presence of allergens in the extract was analyzed by western blotting using pollen allergic patient sera. IgG and IgE epitopes are visualised with anti-human IgG or IgE antibodies.

As shown on figure 1 and 2, there are two major allergens in the extract.

The said crude extract was acidified to pH 3.0 and Tween 20 (0.1%, v/v) was added. This sample constitutes the acidified extract.

### Example 2: Purification of allergen proteins

The allergen extract was purified by:
- Cation exchange chromatography
   A sartobind S⁻ membrane (SARTORIUS) was equilibrated with 28x Bed volume (Bv) of sodium bicarbonate 12.5 mM, citrate 30 mM, pH 3.0, Tween 20 0.1% (v/v). The said acidified extract was loaded on the equilibrated membrane. The column was washed first with 35x Bv of sodium bicarbonate 12.5 mM, citrate 30 mM, pH 3.0, Tween 20 0.1% (v/v) and then washed with 42x Bv of sodium bicarbonate 12.5 mM, citrate 30 mM, pH 3.0. The proteins were eluted with carbonate 0.1 M, sodium chloride 0.5 M, pH 9.15. The presence of proteins was followed the OD at 280 nm. The fractions of interest were pooled.
- Ammonium sulfate precipitation
   This step was performed at 0-4°C.
   A quantity of ammonium sulfate to reach 90% of saturation was added to the product under stirring. The stirring was stopped after the complete dissolution of the salt. The suspension was incubated overnight and centrifuged 2 times during 15 min at 10,000 g. The supernatant was each time carefully discarded.
- Denaturation
   The pellets were resuspended at 9 mg/ml in urea 6 M, DTT 10 mM, Tris.HCl 0.1 M, pH 8.0 and incubated at 37°C for 1 h.
- Size exclusion chromatography on G25 resin (fine Sephadex from AMERSHAM)
   The denatured sample was loaded on the column and the proteins were eluted with Tris.HCl 25 mM, urea 1.5 M, pH 8.0.

The presence of proteins was followed by the OD measurement at 280 nm The fractions of interest were pooled to constitute the purifed denatured allergen extract.

The purified allergen extract was further analysed. The protein content (BCA Assay) and the dry weight were determined in order to evaluate the protein purity. The purification efficiency was also followed by the removal of carbohydrates (Orcinol test) and by the decrease of the ratio OD₂₆₀ / OD₂₈₀.

**Table 1: Removal of non-protein components to form a purified extract**

| | Ratio protein / dry weight | Ratio OD₂₆₀/OD₂₈₀ | Ratio carbohydrates / proteins |
|---|---|---|---|
| Crude extract | 16% | 1.3 | 400% |
| Purified extract | 85% | 0.75 | 17% |

As shown in table 1, the purification process allows
- The increase of the percentage of proteins in the extract from ∼ 15% to 80%
- The OD₂₆₀/OD₂₈₀ ratio to tends towards 0.5 characterizing a pure protein
- A significant removal of carbohydrates (the residual content could represent the carbohydrate moiety of the proteins).

Figure 4 illustrates a typical SDS-PAGE profile obtained for the purified denatured allergen extract. As can be seen, 6 proteins represent at least 60% of the total weight of the proteins in the purified extract.

### Example 3: Hydrolysis of denatured allergen extract

The extract was hydrolyzed using the following protocol :
The said purified allergen extract was acidified to pH 2.0. The digestion was performed at 2.5 mg/ml of pollen proteins and 1 Eu. Ph. U of pepsin (MERCK) for 337 mg of proteins, at 37°C, during 2 h.

Figure 5 shows a comparison between the purified extract (lane 2) and the hydrolyzed extract (lane 3). As can be seen, high molecular weight proteins corresponding to denatured undigested proteins have disappeared after the incubation with pepsin.

### Example 4: Purification

In order to eliminate the peptides with a MW ≥ 10,000 Da and MW ≤ 1,000 Da, the hydrolysate was purified by
- Size exclusion chromatography on G50 resin (fine Sephadex from AMERSHAM)
   16.5 % (v/v) of isopropanol and 0.1 M of NaCl were added to the hydrolysate. This sample was immediately loaded on a G50 column. The peptides were eluted and the fractions containing the peptides (MW ≤ 10 kDa) were pooled as shown in figure 6.
- Diafiltration on 1kDa membrane (ultrafiltation cassette Omega PES from PALL)
   The peptides were concentrated 10 x, diafiltrated against 10 volumes of Tris.HCl 50 mM pH 7.4 and finally concentrated 2.5 x. This sample constitutes the purified allergen hydrolysate.

The efficiency of the purification was controlled by size exclusion HPLC. A BioSep-SEC S2000 column (PHENOMENEX) was equilibrated with Na₂HPO₄ 50 mM - SDS 0.5% (w/v) pH 6.8 at a flow rate of 1 ml/min. The peptides were detected at 214 nm.

The 10 kDa and 1 kDa limits were calculated from a calibration curve as exemplified in figure 7.

As shown on figure 8, peptides with a molecular weight between 1,000 Da and 10,000 Da represent about 75% of all peptides in the purified hydrolysate.

### Example 5: Decrease of allergenicity

Allergenicity properties of the pollen crude extract (according to example 1), purified pollen proteins (according to example 2) and purified pollen peptides (according to example 4) were assessed by measuring their capacity to induce basophile degranulation.

The test was performed *in vitro* on fresh human blood samples from pollen allergic volunteers incubated with increasing concentrations of pollen crude extract, purified proteins and purified peptides. Basophile degranulation was assessed by measuring, by flow cytometric method, the expression of the gp53 protein marker on the cell membrane of activated cells (*i*.*e*. IgE positive cells). This protein is normally present within the membrane of the granules in resting cells and appears on the cell surface upon cell activation (due to the fusion of the granule membrane with the cytoplasmic membrane). It therefore becomes detectable by labeled specific anti-gp53 antibodies. As shown on figure 9, purified peptides are about 30x less allergenic than purified proteins and 100x less allergenic than pollen crude extract.

### Example 6: Effect of adjuvants

To analyze the effect of adjuvants, the immunogenicity of peanut peptides or denatured peanut protein together with different adjuvants was analyzed.

### Treatment of mice

Seven-week-old female naive Balb/c mice were injected subcutaneously, once a week for 6 weeks, with peanut peptides (100 µg) or peanut proteins (25 µg) alone or in combination with different adjuvants: a squalene-based oil-in water emulsion, aluminium hydroxide (500 µg Al³⁺ per injection), aluminium phosphate (500 µg Al³⁺ per injection) and calcium phosphate (200 µg Ca²⁺ per injection). One group of mice receiving a solution of placebo was also included in the study. Blood samples were collected on days 0, 14, 28, 49 and 63 to measure immunoglobulin level induced by treatments.

### Dosage of immunoglobulins G (IgG) specific for peanut proteins in animal serum

Microtiter plates were coated with peanut proteins (2 µg/ml) in carbonate buffer, over night at 4°C, washed and blocked for 1 hour at 37°C with a solution of PBS 0.05% Tween containing a non relevant protein. Wells were incubated with serial dilutions of serum samples (from 1/100 to 1/4374000), for 1 hour at 37°C. Bound IgG were detected with anti-mouse IgG coupled to horseradish peroxydase (incubation of 1 hour at 37°C). After washing, plates were incubated with TMB substrate and the reaction was stopped with 1M H₃PO₄. Absorbance was measured at 450 nm and 650nm.

The results are expressed as titre of peanut-specific IgG which corresponds to inverse of the serum dilution giving an optical density of 0,3.

### Preparation of peanut peptides

Preparation of peanut allergens and hydrolyzed peptides was performed in accordance with the method described in WO 2012/172037, incorporated by reference.

### Results

As shown in Figure 10, a treatment with proteins alone induces peanut specific IgG production (median titre of 150.000 at day 49 after the beginning of the treatment). Addition of aluminium salt adjuvant doubles this production. Calcium phosphate strongly reduces it (median IgG titre of 15.000). Oil-in-water emulsion increases more than seven times the quantity of peanut-specific IgG in treated-mice sera (median IgG titre of 1.100.000).

No production of peanut-specific IgG was observed after 6 injections of 100 µg peanut peptides alone or adsorbed on calcium phosphate and aluminium phosphate (Figure 11). The baseline titre is fixed at a value of 100 which corresponds to the smallest serum dilution. Addition of oil-in water emulsion or aluminium hydroxide in peptide treatments increases the median titre of peanut-specific IgG from day 49 after the beginning of the treatment.

## Claims

1. Allergen preparation comprising an allergen in an oil-in-water emulsion, the allergen preparation comprising squalene, water and one or more surfactants,
wherein the allergen is a hydrolyzed allergen extract, obtainable by a method comprising the steps of
a) extracting a natural source of allergens comprising allergenic proteins to form an extract,
b) purifying of said extract to remove non-protein components to form a purified extract
c) denaturing said purified extract to form a purified denatured extract,
d) refining the purified denatured extract to remove impurities to form a refined denatured extract,
e) hydrolyzing a denatured allergen to form an allergen hydrolysate,
f) optionally purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da
said purified denatured extract comprising proteins, wherein the most abundant (w/w) proteins, forming together at least 60% (w/w) of all proteins, are at least two proteins, and all proteins represent at least 60% (w/w) of the dry weight of the purified denatured extract.

2. The allergen preparation of claim 1 wherein said one or more surfactants is selected from the group consisting of Tween 80, CAMPUL POE-O low PV surfactant, SOLITOL HS15 surfactant, PLURONIC F68 block co-polymer, sodium cholate, glycerodeoxy cholate, sphingomyelin, sphingosine, 1,2-dimyristoyl-sn-glycero-3- phosphoethanolamine, L-a-phosphatidylethanolamine, 1,2-dipalmitoyl-sn-glycero-3phosphocholine and egg phosphatidyl choline, or a mixture thereof.

3. The allergen preparation of any one of claims 1 to 2, wherein the allergen is obtainable by a method comprising the steps of:
a) extracting a source of allergens comprising allergenic proteins to form an extract,
b) purifying the extract to remove non-protein components to form a purified extract,
c) denaturing the purified extract with a first denaturing agent to form a purified denatured extract,
d) refining the purified denatured extract to remove impurities to form a refined denatured extract,
e) denaturing the refined denatured extract with a second denaturing agent to form denatured allergen mixture, and
f) hydrolyzing the denatured allergen mixture to form the hydrolyzed allergens.

4. The allergen preparation of claims 2 or 3 wherein extracting is performed in a solution comprising no salt or a salt selected from carbonate, bicarbonate, phosphate, acetate, TRIS and HEPES.

5. The allergen preparation of any one of claims 2 to 4 wherein the purification of said extract comprises one or more of an ion exchange chromatography step, a gel filtration or size exclusion chromatography step, a precipitation step, a hydrophobic interaction chromatography step, a pseudo affinity or affinity chromatography step or a diafiltration step.

6. The allergen preparation of any one of claims 2 to 5 wherein at least one purification step of said extract is performed with a solution comprising a tenside and/or denaturing agent.

7. The allergen preparation of any one of claims 2 to 6 wherein denaturation is performed with a denaturing agent selected from the group of chaotropic agents, reducing agents and mixtures thereof, preferably among urea, guanidinium chloride, dithiotreitol, thioglycerol, β-mercaptoethanol and mixtures thereof.

8. The allergen preparation of claim 7 wherein the concentration of urea is more than 4 M, preferably more than 5 M and/or the concentration of guanidinium chloride is above 3 M, preferably above 4 M.

9. The allergen preparation of any one of claims 2 to 8 wherein hydrolysing is performed with an enzyme, preferably pepsin, trypsin or chymotrypsin.

10. The allergen preparation of any one of claims 2 to 9 wherein hydrolysing is performed in the presence of a chaotropic agent, preferably selected from urea and guanidinium chloride.

11. The allergen preparation of any one of claims 2 to 10 wherein hydrolyzed allergen is purified to remove peptides with a molecular weight above 10,000 Da and below 1,000 Da, preferably wherein the removal of the peptides is performed by size exclusion chromatography and/or by ultrafiltration.

12. The allergen preparation of claim 11 wherein the size exclusion chromatography step is performed in the presence of a chaotropic agent, preferably selected among urea, guanidinium chloride, ethylene glycol, isopropanol and mixtures thereof.

13. The allergen preparation of any one of claims 1 to 12 wherein the allergens are selected among pollen allergens, milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens including house dust mite, mould allergens, animal allergens, pigeon tick allergens, worm allergens, soft coral allergens, animal dander allergens, nematode allergens, allergens of Hevea brasiliensis.

14. A pharmaceutical product comprising the allergen preparation of any one of claims 1 to 13 or a kit for preparing the allergen preparation of claims 1 to 13 comprising a container of an oil-in-water emulsion and a container comprising a solution of an allergen.

15. The allergen preparation according to any one of claims 1 to 13 for use in the treatment or the prophylaxis of allergy.

## Patentansprüche

1. Allergenpräparat, das ein Allergen in einer Öl-in-Wasser-Emulsion umfasst,
wobei das Allergenpräparat Squalen, Wasser und ein oder mehrere Tenside umfasst,
wobei das Allergen ein hydrolysierter Allergenextrakt ist, der durch ein Verfahren erhältlich ist, das die folgenden Schritte umfasst:
a) Extrahieren einer natürlichen Quelle von Allergenen, die allergene Proteine umfasst, unter Bildung eines Extrakts;
b) Reinigen des Extrakts zur Entfernung von Nichtproteinkomponenten unter Bildung eines gereinigten Extrakts;
c) Denaturieren des gereinigten Extrakts unter Bildung eines gereinigten denaturierten Extrakts;
d) Raffinieren des gereinigten denaturierten Extrakts zur Entfernung von Verunreinigungen unter Bildung eines raffinierten denaturierten Extrakts;
e) Hydrolysieren eines denaturierten Allergens unter Bildung eines Allergenhydrolysats;
f) gegebenenfalls Reinigen des Allergenhydrolysats zur Entfernung von Peptiden mit einem Molekulargewicht von über 10000 Da und unter 1000 Da, um ein gereinigtes Hydrolysat zu erhalten, wobei 70%, besonders bevorzugt 80%, der Peptide zwischen 10 000 Da und 1000 Da liegen;
wobei der gereinigte denaturierte Extrakt Proteine umfasst, wobei die häufigsten (w/w) Proteine, die zusammen wenigstens 60 Gew.-% aller Proteine ausmachen, wenigstens zwei Proteine sind und alle Proteine wenigstens 60 Gew.-% des Trockengewichts des gereinigten denaturierten Extrakts ausmachen.

2. Allergenpräparat gemäß Anspruch 1, wobei das eine oder die mehreren Tenside aus der Gruppe ausgewählt sind, die aus Tween 80, CAMPUL-POE-O-low-PV-Tensid, SOLITOL-HS15-Tensid, PLURONIC-F68-Blockcopolymer, Natriumcholat, Glycerodesoxycholat, Sphingomyelin, Sphingosin, 1,2-Dimyristoyl-sn-glycero-3-phosphoethanolamin, L-a-Phosphatidylethanolamin, 1,2-Dipalmitoyl-sn-glycero-3-phosphocholin und Ei-Phosphatidylcholin oder einem Gemisch davon besteht.

3. Allergenpräparat gemäß einem der Ansprüche 1 bis 2, wobei das Allergen durch ein Verfahren erhältlich ist, das die folgenden Schritte umfasst:
a) Extrahieren einer Quelle von Allergenen, die allergene Proteine umfasst, unter Bildung eines Extrakts;
b) Reinigen des Extrakts zur Entfernung von Nichtproteinkomponenten unter Bildung eines gereinigten Extrakts;
c) Denaturieren des gereinigten Extrakts mit einem ersten Denaturierungsmittel unter Bildung eines gereinigten denaturierten Extrakts;
d) Raffinieren des gereinigten denaturierten Extrakts zur Entfernung von Verunreinigungen unter Bildung eines raffinierten denaturierten Extrakts;
e) Denaturieren des raffinierten denaturierten Extrakts mit einem zweiten Denaturierungsmittel unter Bildung eines denaturierten Allergengemischs; und
f) Hydrolysieren eines denaturierten Allergengemischs unter Bildung der hydrolysierten Allergene.

4. Allergenpräparat gemäß Anspruch 2 oder 3, wobei das Extrahieren in einer Lösung durchgeführt wird, die kein Salz oder ein Salz, das aus Carbonat, Hydrogencarbonat, Phosphat, Acetat, TRIS und HEPES ausgewählt ist, umfasst.

5. Allergenpräparat gemäß einem der Ansprüche 2 bis 4, wobei die Reinigung des Extrakts einen oder mehrere aus einem Ionenaustausch-Chromatographieschritt, einem Gelfiltrations- oder Größenausschluss-Chromatographieschritt, einem Fällungsschritt, einem Hydrophobe-Wechselwirkung-Chromatographieschritt, einem Pseudoaffinitäts- oder Affinitätschromatographieschritt oder einem Diafiltrationsschritt umfasst.

6. Allergenpräparat gemäß einem der Ansprüche 2 bis 5, wobei wenigstens ein Reinigungsschritt des Extrakts mit einer Lösung, die ein Tensid und/oder ein Denaturierungsmittel umfasst, durchgeführt wird.

7. Allergenpräparat gemäß einem der Ansprüche 2 bis 6, wobei die Denaturierung mit einem Denaturierungsmittel durchgeführt wird, das aus der Gruppe der chaotropen Mittel, Reduktionsmittel und Gemische davon, vorzugsweise aus Harnstoff, Guanidiniumchlorid, Dithiothreit, Thioglycerin, β-Mercaptoethanol und Gemischen davon, ausgewählt ist.

8. Allergenpräparat gemäß Anspruch 7, wobei die Konzentration des Harnstoffs mehr als 4 M, vorzugsweise mehr als 5 M, beträgt und/oder die Konzentration von Guanidiniumchlorid über 3 M, vorzugsweise über 4 M, liegt.

9. Allergenpräparat gemäß einem der Ansprüche 2 bis 8, wobei das Hydrolysieren mit einem Enzym, vorzugsweise Pepsin, Trypsin oder Chymotrypsin, durchgeführt wird.

10. Allergenpräparat gemäß einem der Ansprüche 2 bis 9, wobei das Hydrolysieren in Gegenwart eines chaotropen Mittels, das vorzugsweise aus Harnstoff und Guanidiniumchlorid ausgewählt ist, durchgeführt wird.

11. Allergenpräparat gemäß einem der Ansprüche 2 bis 10, wobei das hydrolysierte Allergen zur Entfernung von Peptiden mit einem Molekulargewicht von über 10 000 Da und unter 1000 Da gereinigt wird, wobei vorzugsweise das Entfernen der Peptide durch Größenausschlusschromatographie und/oder durch Ultrafiltration durchgeführt wird.

12. Allergenpräparat gemäß Anspruch 11, wobei der Größenausschluss-Chromatographieschritt in Gegenwart eines chaotropen Mittels, das vorzugsweise aus Harnstoff, Guanidiniumchlorid, Ethylenglycol, Isopropanol und Gemischen davon ausgewählt ist, durchgeführt wird.

13. Allergenpräparat gemäß einem der Ansprüche 1 bis 12, wobei die Allergene aus Pollenallergenen, Milchallergenen, Tiergiftallergenen, Ei-Allergenen, Unkrautallergenen, Grasallergenen, Baumallergenen, Strauchallergenen, Blumenallergenen, Gemüseallergenen, Kornallergenen, Pilzallergenen, Fruchtallergenen, Beerenallergenen, Nussallergenen, Samenallergenen, Bohnenallergenen, Fischallergenen, Schalentierallergenen, Meeresfrüchteallergenen, Fleischallergenen, Gewürzallergenen, Insektenallergenen, Milbenallergenen einschließlich Hausstaubmilben, Schimmelallergenen, Tierallergenen, Taubenzeckenallergenen, Wurmallergenen, Weichkorallenallergenen, Tierhaarallergenen, Nematodenallergenen, Allergenen von *Hevea brasiliensis* ausgewählt sind.

14. Pharmazeutisches Produkt, umfassend das Allergenpräparat gemäß einem der Ansprüche 1 bis 13 oder einen Kit zur Herstellung des Allergenpräparats gemäß Anspruch 1 bis 13, der einen Behälter mit einer Öl-in-Wasser-Emulsion und einen Behälter, der eine Lösung eines Allergens umfasst, umfasst.

15. Allergenpräparat gemäß einem der Ansprüche 1 bis 13 zur Verwendung bei der Behandlung oder Prophylaxe einer Allergie.

## Revendications

1. Préparation allergénique comprenant un allergène dans une émulsion huile-dans-eau,
la préparation allergénique comprenant du squalène, de l'eau et un ou plusieurs tensioactifs,
dans laquelle l'allergène est un extrait d'allergène hydrolysé pouvant être obtenu par un procédé comprenant les étapes consistant à
a) extraire une source naturelle d'allergènes comprenant des protéines allergéniques pour former un extrait,
b) purifier ledit extrait pour éliminer les composants non protéiques afin de former un extrait purifié,
c) dénaturer ledit extrait purifié pour former un extrait purifié dénaturé,
d) épurer l'extrait purifié dénaturé pour éliminer les impuretés afin de former un extrait dénaturé épuré,
e) hydrolyser un allergène dénaturé pour former un hydrolysat allergénique,
f) éventuellement, purifier ledit hydrolysat allergénique pour éliminer les peptides ayant une masse moléculaire supérieure à 10 000 Da et inférieure à 1 000 Da afin d'obtenir un hydrolysat purifié dans lequel 70 %, de manière plus particulièrement préférée 80 % des peptides pèsent entre 10 000 Da et 1 000 Da
ledit extrait purifié dénaturé comprenant des protéines, dans laquelle les protéines les plus abondantes (p/p), constituant ensemble au moins 60 % (p/p) de toutes les protéines, sont au moins deux, et toutes les protéines représentent au moins 60 % (p/p) du poids sec de l'extrait purifié dénaturé.

2. Préparation allergénique selon la revendication 1, dans laquelle ledit un ou plusieurs tensioactifs est choisi dans le groupe formé par le Tween 80, le tensioactif CAMPUL POE-O à faible VP, le tensioactif SOLITOL HS15, le copolymère à blocs PLURONIC F68, le cholate de sodium, le cholate de glycérodésoxy, la sphingomyéline, la sphingosine, la 1,2-dimyristoyl-sn-glycéro-3-phosphoéthanolamine, la L-a-phosphatidyléthanolamine, la 1,2-dipalmitoyl-sn-glycéro-3-phosphocholine et la phosphatidylcholine d'oeuf, ou un mélange de ceux-ci.

3. Préparation allergénique selon l'une quelconque des revendications 1 et 2, dans laquelle l'allergène peut être obtenu par un procédé comprenant les étapes consistant à :
a) extraire une source d'allergènes comprenant des protéines allergéniques pour former un extrait,
b) purifier l'extrait pour éliminer les composants non protéiques afin de former un extrait purifié,
c) dénaturer l'extrait purifié avec un premier agent dénaturant pour former un extrait purifié dénaturé,
d) épurer l'extrait purifié dénaturé pour éliminer les impuretés afin de former un extrait dénaturé épuré,
e) dénaturer l'extrait dénaturé épuré avec un deuxième agent dénaturant pour former un mélange d'allergènes dénaturés, et
f) hydrolyser le mélange d'allergènes dénaturés pour former les allergènes hydrolysés.

4. Préparation allergénique selon les revendications 2 ou 3, dans laquelle l'extraction est réalisée dans une solution ne comprenant aucun sel ou comprenant un sel choisi parmi le carbonate, le bicarbonate, le phosphate, l'acétate, le TRIS et l'HEPES.

5. Préparation allergénique selon l'une quelconque des revendications 2 à 4, dans laquelle la purification dudit extrait comprend une ou plusieurs étapes parmi une étape de chromatographie d'échange d'ions, d'une étape de chromatographie d'exclusion stérique ou de filtration sur gel, une étape de précipitation, une étape de chromatographie d'interactions hydrophobes, une étape de chromatographie d'affinité ou de pseudo-affinité, ou une étape de diafiltration.

6. Préparation allergénique selon l'une quelconque des revendications 2 à 5, dans laquelle au moins une étape de purification dudit extrait est réalisée avec une solution comprenant un tensioactif et/ou un agent dénaturant.

7. Préparation allergénique selon l'une quelconque des revendications 2 à 6, dans laquelle la dénaturation est réalisée avec un agent dénaturant choisi dans le groupe des agents chaotropiques, des agents réducteurs et de leurs mélanges, de préférence parmi l'urée, le chlorure de guanidinium, le dithiotréitol, le thioglycérol, le β-mercaptoéthanol et leurs mélanges.

8. Préparation allergénique selon la revendication 7, dans laquelle la concentration en urée est supérieure à 4 M, de préférence supérieure à 5 M et/ou la concentration en chlorure de guanidinium est supérieure à 3 M, de préférence supérieure à 4 M.

9. Préparation allergénique selon l'une quelconque des revendications 2 à 8, dans laquelle l'hydrolyse est réalisée avec une enzyme, de préférence la pepsine, la trypsine ou la chymotrypsine.

10. Préparation allergénique selon l'une quelconque des revendications 2 à 9, dans laquelle l'hydrolyse est réalisée en présence d'un agent chaotropique de préférence choisi parmi l'urée et le chlorure de guanidinium.

11. Préparation allergénique selon l'une quelconque des revendications 2 à 10, dans laquelle l'allergène hydrolysé est purifié pour éliminer les peptides ayant une masse moléculaire supérieure à 10 000 Da et inférieure à 1 000 Da, de préférence dans laquelle l'élimination des peptides est réalisée par chromatographie d'exclusion stérique et/ou par ultrafiltration.

12. Préparation allergénique selon la revendication 11, dans laquelle l'étape de chromatographie d'exclusion stérique est réalisée en présence d'un agent chaotropique de préférence choisi parmi l'urée, le chlorure de guanidinium, l'éthylèneglycol, l'isopropanol et les mélanges de ceux-ci.

13. Préparation allergénique selon l'une quelconque des revendications 1 à 12, dans laquelle les allergènes sont choisis parmi les allergènes de pollens, les allergènes du lait, les allergènes de venins, les allergènes de l'oeuf, les allergènes d'herbacées, les allergènes de graminées, les allergènes d'arbres, les allergènes d'arbustes, les allergènes de fleurs, les allergènes de légumes, les allergènes de céréales, les allergènes de champignons, les allergènes de fruits, les allergènes de baies, les allergènes de fruits à coque, les allergènes de graines, les allergènes de légumineuses, les allergènes de poissons, les allergènes de mollusques, les allergènes de crustacés, les allergènes de la viande, les allergènes d'épices, les allergènes d'insectes, les allergènes d'acariens comprenant les allergènes d'acariens de la poussière de maison, les allergènes de moisissure, les allergènes d'animaux, les allergènes de tiques de pigeon, les allergènes de vers, les allergènes de coraux mous, les allergènes de squames d'animaux, les allergènes de nématodes, les allergènes *d'Hevea brasiliensis.*

14. Produit pharmaceutique comprenant la préparation allergénique selon l'une quelconque des revendications 1 à 13 ou trousse pour préparer la préparation allergénique selon les revendications 1 à 13 comprenant un récipient d'une émulsion huile-dans-eau et un récipient contenant une solution d'un allergène.

15. Préparation allergénique selon l'une quelconque des revendications 1 à 13, pour une utilisation dans le traitement ou la prophylaxie des allergies.
